# EUROPEAN PATENT APPLICATION

(11) **EP 4 810 075 A1**
(43) Date of publication of application: **23.09.2026**
(21) Application number: 24890333.8
(22) Date of filing: 23.09.2024
(51) Int. Cl.: A61M 1/06

(54) **SELF-ADSORPTION MILK PUMPING APPARATUS AND BREAST PUMP**

(30) Priority: 14.11.2023 CN 202323080630 U; 03.06.2024 CN 202410710475; 03.06.2024 CN 202421253081 U
(71) Applicant: Ningbo Xiaowu Brand Management Co., Ltd., Zhejiang 315000 (CN)
(72) Inventor: YE, Qiansheng, Ningbo, Zhejiang 315000 (CN); WANG, Yongliang, Ningbo, Zhejiang 315000 (CN)
(74) Representative: Metida
(86) International application number: PCT/CN2024/120258
(87) International publication number: WO 2025/102975

(57) **Abstract**

A self-adsorption milk pumping apparatus (100). The self-adsorption milk pumping apparatus (100) comprises a one-way valve (30), a self-adsorption shield (20), and a negative pressure forming unit (10). The negative pressure forming unit (10) is provided with a negative pressure cavity (13), a gas exchange channel (111), a milk pumping cavity (121), an assembly opening (122), and a milk discharge channel (123). The gas exchange channel (111) is in communication with the negative pressure cavity (13). The assembly opening (122) and the milk discharge channel (123) are respectively in communication with the milk pumping cavity (121). The milk pumping cavity (121) and the negative pressure cavity (13) are separated by a deformable component (125) of the negative pressure forming unit (10). The self-adsorption shield (20) is mounted to the assembly opening (122) of the negative pressure forming unit (10). The one-way valve (30) is mounted to the negative pressure forming unit (10) to selectively open and close the milk discharge channel (123). The self-adsorption shield (20) can be adsorbed onto the breast, so that the self-adsorption milk pumping apparatus (100) is adsorbed onto the breast, thereby assisting a user in pumping milk.

## Description

### BACKGROUND OF THE PRESENT INVENTION

### FIELD OF INVENTION

The present invention relates to a breast pump, belonging to the technical field of maternal and infant products, and more particularly to a self-adsorption milk pumping apparatus and a breast pump.

### DESCRIPTION OF RELATED ARTS

A wearable breast pump is a type of breast pump that can be placed inside a bra, wherein the bra is used to secure the breast pump to the breast. In this way, the mother's hands can be freed during the pumping process, and the mother can even move around freely. Therefore, wearable breast pumps have become increasingly popular in recent years. However, existing wearable breast pumps have many defects. For example, first, without the assistance of a bra, the wearable breast pump cannot be fixed at the breast position; second, after the wearable breast pump is fixed to the breast by means of the bra, the wearable breast pump supports the bra toward the front, causing the side portions of the bra to press against the side of the breast and the back, thereby affecting comfort; third, the breast shield of the wearable breast pump is integrally fitted to the main unit, and the angle of the breast shield is always consistent with the angle of the main unit, so the mother needs to adjust the angle of the main unit to change the angle of the breast shield. Due to the weight of the main unit, and as pumping proceeds, the weight of the collected milk acts on the main unit and further increases its weight, causing the main unit to slide downward. The downward sliding of the main unit inevitably causes the angle of the breast shield to change and become misaligned, or even detach from the breast, thereby resulting in milk leakage. How to enable the breast pump to be fixed at the breast position without requiring any auxiliary support is the technical problem that the inventor of the present invention is committed to solving.

### SUMMARY OF THE PRESENT INVENTION

An object of the present invention is to provide a self-adsorption milk pumping apparatus and a breast pump, wherein the self-adsorption milk pumping apparatus can be adsorbed onto the breast without any assistance.

An object of the present invention is to provide a self-adsorption milk pumping apparatus and a breast pump, wherein during the breast pumping process, the self-adsorption cover of the self-adsorption milk pumping apparatus can reliably attach to the breast, thereby allowing the self-adsorption milk pumping apparatus to be attached to the breast without any assistance.

An object of the present invention is to provide a self-adsorption milk pumping apparatus and a breast pump, wherein the center of gravity of the self-adsorption milk pumping apparatus is close to the self-adsorption cover, so that when the self-adsorption cover is adsorbed onto the breast, the center of gravity of the self-adsorption milk pumping apparatus is closer to the body of the user. In this way, the self-adsorption milk pumping apparatus can reduce the adsorption force required on the self-adsorption cover, thereby avoiding the self-adsorption milk pumping apparatus from compressing the breast during the breast pumping process, thus ensuring breast health.

An object of the present invention is to provide a self-adsorption milk pumping apparatus and a breast pump, wherein after the horn cover of the self-adsorption cover is fitted against the breast to adsorb the self-adsorption milk pumping apparatus onto the breast, when the assembly ring of the self-adsorption cover changes with the position of the negative pressure forming unit, the shoulder ring of the self-adsorption cover can deform to prevent the horn cover from changing with the position of the negative pressure forming unit, so that the self-adsorption cover can reliably attach to the breast during the breast pumping process.

An object of the present invention is to provide a self-adsorption milk pumping apparatus and a breast pump, wherein the self-adsorption cover is detachable, so that, on the one hand, the self-adsorption cover can be cleaned separately to improve the hygiene of the self-adsorption milk pumping apparatus, and on the other hand, mothers can choose the appropriate self-adsorption cover as needed to meet their individual needs.

An object of the present invention is to provide a self-adsorption milk pumping apparatus and a breast pump, wherein when the self-adsorption cover is installed on the negative pressure forming unit, the self-adsorption cover can be limited to a gap between the shoulder ring and the negative pressure forming unit, so that the shoulder ring can deform when the assembly ring changes position with the negative pressure forming unit. Furthermore, by limiting the self-adsorption cover, the assembly ring can be prevented from obstructing the milk discharge channel of the negative pressure forming unit, thus facilitating smooth milk discharge.

An object of the present invention is to provide a self-adsorption milk pumping apparatus and a breast pump, wherein the self-adsorption milk pumping apparatus has a small size, so that after the self-adsorption milk pumping apparatus is adsorbed onto the breast, even if a bra needs to be worn, the self-adsorption milk pumping apparatus will not excessively push the bra outwards. At the same time, the pressure exerted by the bra on the breast through the self-adsorption milk pumping apparatus will also be reduced, thereby improving the comfort of the mother when pumping milk through the self-adsorption milk pumping apparatus.

According to one aspect of the present invention, the present invention provides self-adsorption milk pumping apparatus, comprising:
a one-way valve;
a self-adsorption cover; and
a negative pressure forming unit having a negative pressure chamber, an air exchange channel, a milk suction chamber, an assembly opening, and a milk discharge channel, wherein the air exchange channel is communicated to the negative pressure chamber, the assembly opening and the milk discharge channel are respectively communicated to the milk suction chamber, the negative pressure forming unit comprises a deformable component, and the milk suction chamber and the negative pressure chamber are separated by the deformable component of the negative pressure forming unit, wherein the self-adsorption cover is installed in the assembly opening of the negative pressure forming unit, and the one-way valve is provided in the negative pressure forming unit to selectively open and close the milk discharge channel.

According to an embodiment of the present invention, the self-adsorption cover has a horn cover which is deformable, and from a side view, an active space is formed between the horn cover and the negative pressure forming unit to allow the horn cover to flip towards the negative pressure forming unit.

According to an embodiment of the present invention, the self-adsorption cover comprises a horn cover which is deformable and an abutment portion, the horn cover is arranged to cover a portion of a breast, the abutment portion is arranged to abut an areola, wherein a distance D1 between the abutment portion and an outer edge of the horn cover is greater than a distance D2 between a portion of the negative pressure forming unit arranged to define the assembly opening and the outer edge of the horn cover.

According to an embodiment of the present invention, the self-adsorption cover comprises an abutment portion for abutting an areola, wherein a distance D1 between the abutment portion and an outer edge of the horn cover is greater than a distance D2 between a portion of the negative pressure forming unit arranged to define the assembly opening and the outer edge of the horn cover.

According to an embodiment of the present invention, the self-adsorption cover comprises a shoulder ring, an assembly ring, and a horn cover which is deformable, wherein the assembly ring is extended integrally to one side from an inner edge of the shoulder ring, and the horn cover is extended integrally in an opposite direction from an outer edge of the shoulder ring.

According to an embodiment of the present invention, the self-adsorption cover further comprises a shoulder ring and an assembly ring, wherein the assembly ring is extended integrally to one side from an inner edge of the shoulder ring, and the horn cover is extended integrally in an opposite direction from an outer edge of the shoulder ring.

According to an embodiment of the present invention, the self-adsorption cover comprises an abutment portion located at a connection between the assembly ring and the shoulder ring for abutting an areola, wherein a distance D1 between the abutment portion and an outer edge of the horn cover is greater than a distance D2 between a portion of the negative pressure forming unit arranged to define the assembly opening and the outer edge of the horn cover.

According to an embodiment of the present invention, the self-adsorption cover comprises a stabilizing part which is disposed at a connection position between the shoulder ring and the horn cover to maintain a shape of the horn cover.

According to an embodiment of the present invention, the horn cover is flexible, while the shoulder ring and the assembly ring are rigid.

According to an embodiment of the present invention, the self-adsorption cover comprises a stabilizing part disposed on the outside of the assembly ring and the shoulder ring to maintain the shape of the horn cover.

According to an embodiment of the present invention, the self-adsorption cover is extended through the assembly opening of the negative pressure forming unit to an outside of the milk discharge channel to form a step.

According to an embodiment of the present invention, the shoulder ring is extended outward and forward from the assembly ring in a direction substantially perpendicular to the assembly ring, wherein the horn cover is extended outward and forward from the shoulder ring in a direction substantially horizontal to the assembly ring.

According to an embodiment of the present invention, the self-adsorption cover comprises a limiting portion that is extended integrally from a peripheral wall of the assembly ring in a direction perpendicular to an extending direction of the assembly ring, and the limiting portion is spaced apart from the shoulder ring.

According to an embodiment of the present invention, a distance d1 between an outer side of the limiting portion and a central axis of the self-adsorption cover is greater than a distance d2 between an outer side of the shoulder ring and the central axis of the self-adsorption cover.

According to an embodiment of the present invention,a hardness of the horn cover of the self-adsorption cover is in the range of 10A to 50A.

According to an embodiment of the present invention, a projected length L of the horn cover of the self-adsorption cover in a horizontal plane ranges from 12 mm to 30 mm.

According to an embodiment of the present invention,a surface roughness of an inner wall of the horn cover ranges from Ra0.016um to Ra0.063um.

According to an embodiment of the present invention, an angle α between an extending direction of an inner wall of the horn cover of the self-adsorption cover and a central axis of the self-adsorption cover ranges from 15° to 40°.

According to an embodiment of the present invention, an extension direction of an inner wall of the horn cover of the self-adsorption cover and a central axis of the self-adsorption cover define an angle α, and an extension direction of an inner wall of the shoulder ring and the central axis of the self-adsorption cover define an angle β, wherein the angle β is greater than the angle α.

According to an embodiment of the present invention, the negative pressure forming unit comprises a supporting housing and a negative pressure forming part which is installed on the supporting housing, wherein the negative pressure chamber is formed between the supporting housing and the negative pressure forming part, the air exchange channel is provided on the supporting housing, the milk suction chamber, the assembly opening and the milk discharge channel are provided on the negative pressure forming part, wherein the self-adsorption cover and the one-way valve are respectively installed on the negative pressure forming part.

According to an embodiment of the present invention, the supporting housing comprises a housing body and a horn body integrally extended outward from one end of the housing body, wherein the housing body has a mounting cavity and a mounting opening communicated with the mounting cavity, wherein the negative pressure forming part comprises a deformable portion and a frame portion, the deformable portion has a connecting end and a deformable end opposite to the connecting end, the milk suction chamber, the assembly opening, and the milk discharge channel are formed in the deformable portion, the frame portion is connected to the connecting end of the deformable portion, the frame portion of the negative pressure forming part is mounted to the mounting opening of the housing body, the deformable portion is extended through the mounting opening to the mounting cavity, the negative pressure chamber is formed between the deformable portion and the housing body, the deformable portion is the deformable component of the negative pressure forming unit for separating the negative pressure chamber and the milk suction chamber.

According to another aspect of the present invention, the present invention further provides a breast pump comprising:
a breast pump main unit;
at least one air tube; and
at least one self-adsorption milk pumping apparatus comprising a one-way valve, a self-adsorption cover, and a negative pressure forming unit, wherein the negative pressure forming unit has a negative pressure chamber, an air exchange channel, a milk suction chamber, an assembly opening, and a milk discharge channel, wherein the air exchange channel is connected to the negative pressure chamber, the assembly opening and the milk discharge channel are respectively communicated to the milk suction chamber, the milk suction chamber and the negative pressure chamber are separated by a deformable component of the negative pressure forming unit, wherein the self-adsorption cover is installed in the assembly opening of the negative pressure forming unit, wherein the one-way valve is installed in the negative pressure forming unit to selectively open and close the milk discharge channel, wherein the air tube connects the breast pump main unit and the air exchange channel of the negative pressure forming unit.

### BRIEF DESCRIPTION OF THE DRAWINGS

FIG. 1 is a perspective view of a breast pump according to a preferred embodiment of the present invention.
FIG. 2 is a schematic view of the use state of the breast pump according to the above mentioned preferred embodiment of the present invention.
FIG. 3 is a perspective view of a self-adsorption milk pumping apparatus of the breast pump according to the above mentioned preferred embodiment of the present invention.
FIG. 4 is another perspective view of the self-adsorption milk pumping apparatus of the breast pump according to the above mentioned preferred embodiment of the present invention.
FIG. 5 is an exploded view of the self-adsorption milk pumping apparatus of the breast pump according to the above mentioned preferred embodiment of the present invention.
FIG. 6 is another exploded view of the self-adsorption milk pumping apparatus of the breast pump according to the above mentioned preferred embodiment of the present invention.
FIG. 7 is a perspective cross-sectional view of the self-adsorption milk pumping apparatus of the breast pump according to the above mentioned preferred embodiment of the present invention.
FIG. 8 is a cross-sectional view of the self-adsorption milk pumping apparatus of the breast pump according to the above mentioned preferred embodiment of the present invention from a plan view perspective.
FIG. 9 is an enlarged view of a partial location in FIG. 8.
FIG. 10 is a cross-sectional view from another planar perspective of the self-adsorption milk pumping apparatus of the breast pump according to the above mentioned preferred embodiment of the present invention.
FIG. 11 is an enlarged view of a partial location in FIG. 10.
FIG. 12 is a schematic view illustrating the assembly process from a cross-sectional perspective of the self-adsorption milk pumping apparatus of the breast pump according to the above mentioned preferred embodiment of the present invention.
FIG. 13 is a schematic view illustrating the assembly process from another cross-sectional perspective of the self-adsorption milk pumping apparatus of the breast pump according to the above mentioned preferred embodiment of the present invention.
FIG. 14 is a cross-sectional schematic view of a first alternative mode of the self-adsorption milk pumping apparatus of the breast pump according to the above mentioned preferred embodiment of the present invention.
FIG. 15 is a cross-sectional schematic view of a second alternative mode of the self-adsorption milk pumping apparatus of the breast pump according to the above mentioned preferred embodiment of the present invention.
FIG. 16 is a cross-sectional schematic view of a third alternative mode of the self-adsorption milk pumping apparatus of the breast pump according to the above mentioned preferred embodiment of the present invention.
FIG. 17 is a cross-sectional schematic view of a fourth alternative mode of the self-adsorption milk pumping apparatus of the breast pump according to the above mentioned preferred embodiment of the present invention.

### DETAILED DESCRIPTION OF THE PREFERRED EMBODIMENT

Before detailing any embodiment of the invention, it should be understood that the invention, in its application, is not limited to the details of the construction and arrangement of the components set forth in the following description or illustrated in the following figures. The invention can have other embodiments and can be practiced or carried out in various ways. Furthermore, it should be understood that the wording and terminology used herein are for descriptive purposes and should not be considered limiting. The use of "comprising" or "having" and variations thereof is intended to cover the items set forth below and their equivalents, as well as any additional items. Unless otherwise specified or limited, the terms "installation", "connection", "support", and "linkage", and variations thereof are used broadly and cover both direct and indirect installation, connection, support, and linking. Moreover, "connect" and "link" are not limited to physical or mechanical connections or links.

Furthermore, firstly, in the disclosure of the present invention, the terms "longitudinal", "lateral", "upper", "lower", "front", "rear", "left", "right", "vertical", "horizontal", "top", "bottom", "inner", and "outer", etc., indicate the orientation or positional relationship based on the orientation or positional relationship shown in the accompanying drawings. They are only for the convenience of describing the present invention and simplifying the description, and do not indicate or imply that the device or element referred to must have a specific orientation, or be constructed and operated in a specific orientation. Therefore, the above terms should not be construed as limiting the invention. Secondly, the term "a" or "an" should be understood as "at least one" or "one or more", that is, in one embodiment, the number of an element can be one, while in another embodiment, the number of the element can be multiple. The term "a" or "an" should not be construed as a limitation on the quantity.

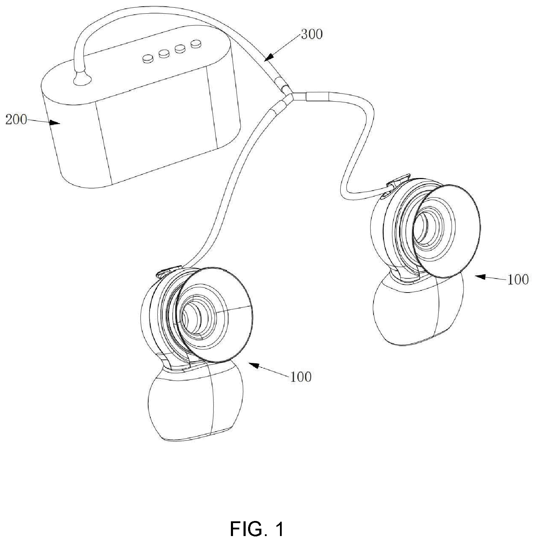

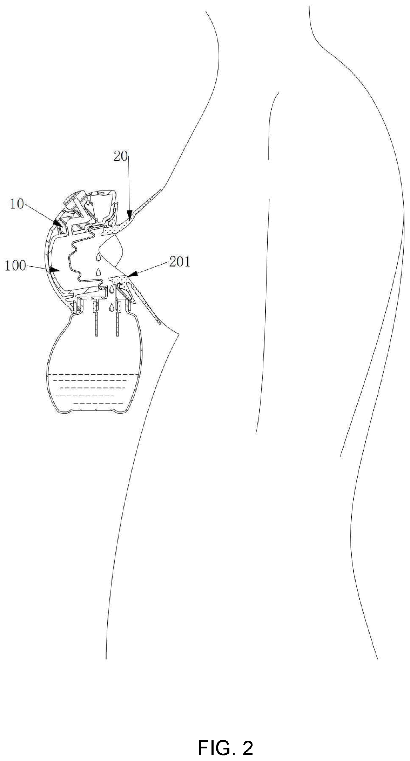

Referring to FIGS. 1 and 2 of the accompanying drawings of the present invention, a breast pump according to a preferred embodiment of the present invention will be disclosed and described below. The breast pump comprises at least one self-adsorption milk pumping apparatus 100, a breast pump main unit 200, and at least one air tube 300. The two opposite ends of the air tube 300 are respectively connected to the self-adsorption milk pumping apparatus 100 and the breast pump main unit 200. The self-adsorption milk pumping apparatus 100 can be adsorbed onto and attached to the breast, and when the breast pump main unit 200 is in operation, the self-adsorption milk pumping apparatus 100 assists the mother in expressing milk. In the breast pump of the present invention, the breast pump allows the self-adsorption milk pumping apparatus 100 to self-attach to the breast, that is, the self-adsorption milk pumping apparatus 100 can be adsorbed onto the breast without any assistance, for example, without wearing a bra. It is understood that the mother can also wear a bra after the self-adsorption milk pumping apparatus 100 is self-attached onto the breast.

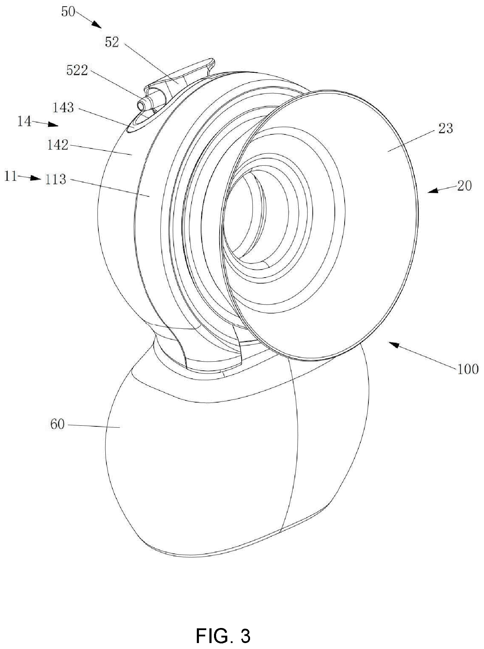

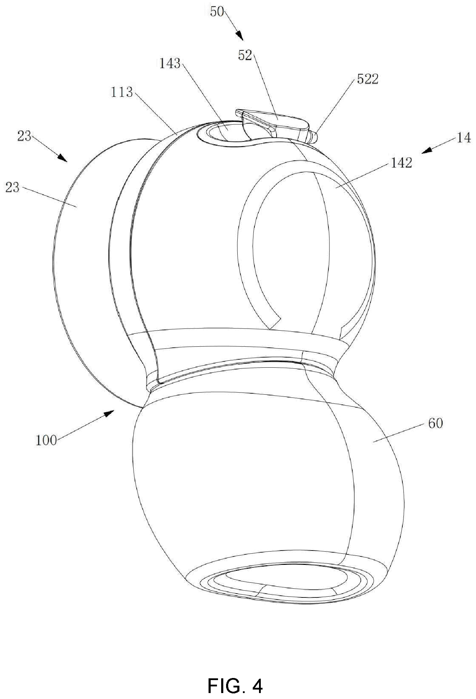

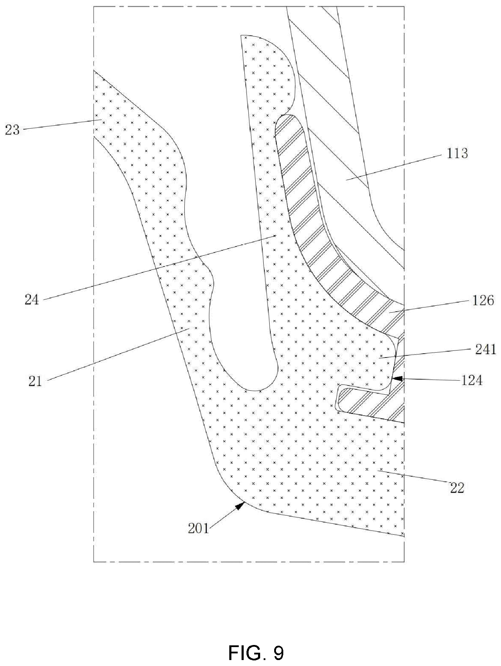

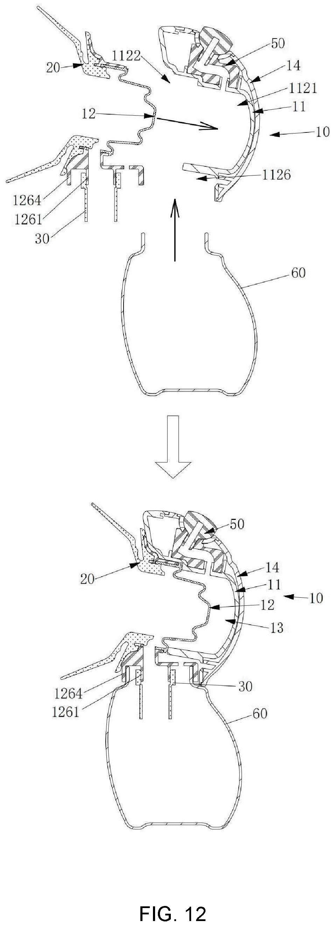

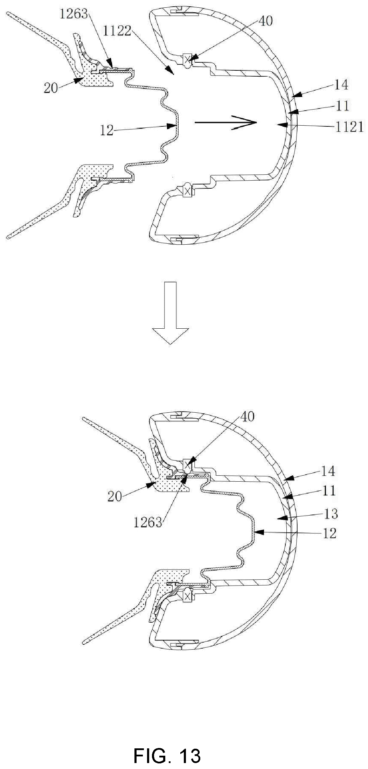

FIGS. 3 to 13 show a specific example of the self-adsorption type breast pumping device 100 which comprises a negative pressure forming unit 10, a self-adsorption cover 20 assembled in the negative pressure forming unit 10, and a one-way valve 30 assembled in the negative pressure forming unit 10.

Specifically, the negative pressure forming unit 10 comprises a supporting housing 11 and a negative pressure forming part 12, and has a negative pressure chamber 13. The negative pressure forming part 12 is installed on the supporting housing 11, and the negative pressure chamber 13 is formed between the supporting housing 11 and the negative pressure forming part 12. The supporting housing 11 has an air exchange channel 111 which is communicated to the negative pressure chamber 13. The negative pressure forming part 12 has a milk suction chamber 121, and an assembly opening 122 and a milk discharge channel 123 which are respectively communicated to the milk suction chamber 121. The negative pressure chamber 13 and the milk suction chamber 121 are independent of each other, that is, the negative pressure chamber 13 and the milk suction chamber 121 are separated. The self-adsorption cover 20 is assembled to the assembly opening 122 of the negative pressure forming part 12. The one-way valve 30 is disposed on the negative pressure forming part 12, and the one-way valve 30 selectively opens and closes the milk discharge channel 123. The self-adsorption cover 20 can attach to the breast, thereby allowing the self-adsorption milk pumping apparatus 100 to be attached to the breast.

In this specific example of the self-adsorption milk pumping apparatus 100 shown in FIGS. 3 to 13, the self-adsorption cover 20 has an abutment portion 201 for abutting the areola.

The air exchange channel 111 of the supporting housing 11 can be connected to the breast pump main unit 200 through the air tube 300. When the mother attaches the self-adsorption milk pumping apparatus 100 to the breast by attaching it to the breast through the self-adsorption cover 20, the areola part abuts against the abutment portion 201 of the self-adsorption cover 20, so that the self-adsorption cover 20 covers the nipple. In this way, the areola part and the one-way valve 30 can form a closed space in the milk suction chamber 121 of the negative pressure forming part 12. When the breast pump main unit 200 is in operation, the air in the negative pressure chamber 13 of the negative pressure forming unit 10 can be discharged through the air exchange channel 111 of the supporting housing 11 and the air tube 300 to reduce the pressure in the negative pressure chamber 13. At this time, the one-way valve 30 closes the milk discharge channel 123 of the negative pressure forming part 12, and the space of the milk suction chamber 121 of the negative pressure forming part 12 increases, resulting in a decrease in the pressure of the milk suction chamber 121. At this time, milk can be sucked out. During this process, the areola tends to be pulled towards the milk suction chamber 121 of the negative pressure forming part 12. Since the areola is against the abutment portion 201 of the self-adsorption cover 20, the abutment portion 201 of the self-adsorption cover 20 prevents the areola from being pulled toward the milk suction chamber 121 of the negative pressure forming part 12, so that the areola is squeezed and the milk is squeezed out. In this way, the milk suction efficiency can be improved. Subsequently, ambient air enters the negative pressure chamber 13 of the negative pressure forming unit 10 through the air tube 300 and the air exchange channel 111 of the supporting housing 11. The pressure in the negative pressure chamber 13 increases, at which point the one-way valve 30 opens the milk discharge channel 123 of the negative pressure forming part 12 to discharge milk. The space of the milk suction chamber 121 in the negative pressure forming part 12 decreases, causing the pressure in the milk suction chamber 121 to increase. By alternately lowering and raising the pressure in the negative pressure chamber 13 of the negative pressure forming unit 10, the breast pump can achieve milk suction. That is, the negative pressure chamber 13 and the milk suction chamber 121 are separated by the deformable portion of the negative pressure forming unit 10, so the pressure in the milk suction chamber 121 changes with the pressure in the negative pressure chamber 13.

As shown in FIGS. 3 to 13, the self-adsorption cover 20 comprises a shoulder ring 21, an assembly ring 22, and a deformable horn cover 23. The assembly ring 22 is extended to one side from the inner edge of the shoulder ring 21, and the horn cover 23 is extended in the opposite direction from the outer edge of the shoulder ring 21. The abutment portion 201 of the self-adsorption cover 20 is located at the connection between the shoulder ring 21 and the assembly ring 22. Preferably, in a specific example of the self-adsorption milk pumping apparatus 100 of the present invention, the assembly ring 22 is extended integrally to one side from the inner edge of the shoulder ring 21, and the horn cover 23 is extended integrally in the opposite direction from the outer edge of the shoulder ring 21, that is, the self-adsorption cover 20 is a single unit. For example, in some embodiments, the self-adsorption cover 20 can be injection molded from silicone material, thus making the self-adsorption cover 20 flexible and a single unit.

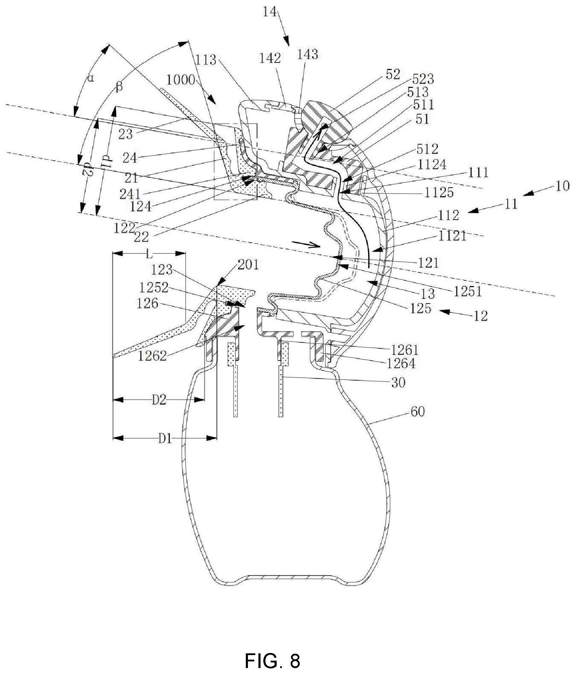

Referring to FIG. 8, the assembly ring 22 of the self-adsorption cover 20 is assembled into the assembly opening 122 of the negative pressure forming part 12, and there is an active space 1000 between the horn cover 23 and the negative pressure forming unit 10 to allow the deformed horn cover 23 to flip onto the negative pressure forming unit 10. When the mother attaches the self-adsorption milk pumping apparatus 100 to her breast via the self-adsorption cover 20, the breast closes the opening of the horn cover 23. This allows the mother to: 1) flip the horn cover 23 toward the negative pressure forming unit 10 before attaching the self-adsorption milk pumping apparatus 100; 2) align the nipple with the milk suction chamber 121 of the negative pressure forming part 12 and the abutment portion 201 of the self-adsorption cover 20 against the areola; and 3) retract the horn cover 23, ensuring a tight fit against the breast. This allows the breast to close the opening of the horn cover 23, enabling the self-adsorption cover 20 to attach to the breast, while the abutment portion 201 of the self-adsorption cover 20 remains in contact with the areola. Thus, during breast pumping, the milk suction chamber 121 of the negative pressure forming part 12 and the self-adsorption cover 20 maintain contact with the areola. A portion of the internal space of the self-adsorption cover 20 forms a negative pressure environment. Furthermore, after the mother attaches the self-adsorption milk pumping apparatus 100 to her breast via the self-adsorption cover 20, the self-adsorption cover 20 isolates the negative pressure forming unit 10 from the breast, preventing the negative pressure forming unit 10 from directly contacting the breast. The flexible self-adsorption cover 20 improves the mother's comfort when using the breast pump. On the other hand, when the assembly ring 22 of the self-adsorption cover 20 changes position with the negative pressure forming unit 10, the shoulder ring 21 of the self-adsorption cover 20 can deform to prevent the horn cover 23 from changing position with the negative pressure forming unit 10. Thus, during breast pumping, the self-adsorption cover 20 can always reliably fit the mother's breast, preventing the self-adsorption milk pumping apparatus 100 from falling off the breast and preventing milk leakage.

In the self-adsorption milk pumping apparatus 100 of the present invention, referring to FIG. 8, the distance D1 between the abutment portion 201 of the self-adsorption cover 20 and the outer edge of the horn cover 23 is greater than the distance D2 between the portion of the negative pressure forming part 12 used to define the assembly opening 122 and the outer edge of the horn cover 23, so that the abutment portion 201 of the self-adsorption cover 20 is retracted. In this way, the center of gravity of the self-adsorption milk pumping apparatus 100 can be closer to the self-adsorption cover 20. Therefore, when the self-adsorption cover 20 is adsorbed onto the breast, the center of gravity of the self-adsorption milk pumping apparatus 100 is closer to the body. Thus, the component of gravity of the self-adsorption milk pumping apparatus 100 acting on the breast can be reduced, thereby reducing the adsorption force required by the self-adsorption cover 20 to prevent the self-adsorption milk pumping apparatus 100 from falling off the breast and to prevent the self-adsorption milk pumping apparatus 100 from compressing the breast during breast pumping, thereby ensuring breast health.

In the self-adsorption milk pumping apparatus 100 of the present invention, referring to FIG. 8, the shoulder ring 21 is extended outward and forward from the assembly ring 22 in a direction substantially perpendicular to the assembly ring 22, and the horn cover 23 is extended outward and forward from the shoulder ring 21 in a direction substantially horizontal to the assembly ring 22. Thus, with a small distance between the outer edge of the horn cover 23 and the outer edge of the assembly ring 22, the outer edge of the horn cover 23 can form a larger opening, and the self-adsorption cover 20 can form a smaller opening at the position of the abutment portion 201. This allows the horn-shaped cover to attach to the breast and the abutment portion 201 to contact the areola. When the self-adsorption cover 20 adheres to the breast, the center of gravity of the self-adsorption milk pumping apparatus 100 is closer to the body, reducing the component of gravity acting on the breast. This lowers the suction force required by the self-adsorption cover 20, preventing the self-adsorption milk pumping apparatus 100 from falling off the breast and avoiding pressure on the breast during pumping, thus ensuring breast health.

Furthermore, in the self-adsorption milk pumping apparatus 100 of the present invention, the negative pressure forming unit 10 of the self-adsorption milk pumping apparatus 100 does not need to be equipped with any electronic components. Instead, the air exchange channel 111 of the supporting housing 11 of the negative pressure forming unit 10 and the breast pump main unit 200 are connected through an air tube 300. In this way, the self-adsorption milk pumping apparatus 100 can have a lighter weight and a smaller size. This not only allows the center of gravity of the self-adsorption milk pumping apparatus 100 to be closer to the self-adsorption cover 20, so that the self-adsorption milk pumping apparatus 100 can be reliably adsorbed on the breast, but also reduces the burden on the breast due to the lighter weight.

Referring again to FIG. 8, the inner wall of the horn cover 23 of the self-adsorption cover 20 is an inclined surface, and its extending direction forms an angle α with the central axis of the self-adsorption cover 20. This angle α cannot be too small or too large. If the angle α is too small, the opening size of the horn cover 23 will be too small, which will prevent the horn cover 23 from covering the breast and the contact part 23 of the self-adsorption cover 20 from contacting the areola. Even if the breast is forcibly inserted into the horn cover 23, the horn cover 23 will provide a large clamping force and act on the breast, causing breast discomfort or even damage. If the angle α is too large, the opening size of the horn cover 23 will be too large, which will prevent the horn cover 23 from fitting the breast and prevent the self-adsorption cover 20 from attaching to the breast. In the self-adsorption type breast pumping device 100 of the present invention, the minimum value of the comprised angle α is preferably not less than 15° and the maximum value is preferably not greater than 40°. Therefore, the suitable range of the comprised angle α is 15° to 40°.

Furthermore, referring to FIG. 8, the inner wall of the shoulder ring 21 of the self-adsorption cover 20 is an inclined surface, and its extending direction forms an angle β with the central axis of the self-adsorption cover 20. This angle β is greater than the angle α. Thus, when the outer edge of the horn cover 23 and the outer edge of the assembly ring 22 are relatively close, the outer edge of the horn cover 23 can form a larger opening, while the self-adsorption cover 20 can form a smaller opening at the abutment portion 201, thereby enabling the horn cover 23 to attach to and absorbed onto the breast while the abutment portion 201 is biasing against the areola. When the self-adsorption cover 20 is attached to the breast, the center of gravity of the self-adsorption milk pumping apparatus 100 is closer to the body of the user. The component of gravity of the self-adsorption milk pumping apparatus 100 acting on the breast can be reduced, thereby reducing the suction force required by the self-adsorption cover 20 to prevent the self-adsorption milk pumping apparatus 100 from falling off the breast and to prevent the self-adsorption milk pumping apparatus 100 from compressing the breast during breast pumping, thus ensuring breast health.

Referring again to FIG. 8, the projected length L of the horn cover 23 of the self-adsorption cover 20 in the horizontal plane cannot be too large or too small. If the projected length L is too small, the contact area between the horn cover 23 and the breast will be too small, resulting in the self-adsorption cover 20 not being able to reliably adhere to the breast. If the projected length L is too large, the weight of the self-adsorption milk pumping apparatus 100 will increase, putting a burden on the breast. In the self-adsorption milk pumping apparatus 100 of the present invention, the minimum value of the projected length L is preferably not less than 12 mm, and the maximum value is preferably not greater than 30 mm. Therefore, the suitable range of the projected length L is 12 mm to 30 mm.

Furthermore, the hardness range of the horn cover 23 of the self-adsorption cover 20 is from 10A to 50A, so as to improve comfort while ensuring the flexibility of the horn cover 23. The surface roughness range of the inner wall of the horn cover 23 of the self-adsorption cover 20 is Ra0.016um-Ra0.063um, so that when the horn cover 23 is adsorbed onto the breast, there is a reasonable frictional force between the horn cover 23 and the breast, so that the self-adsorption cover 20 can stably attach to the breast.

In one example of the self-adsorption milk pumping apparatus 100 shown in FIGS. 3 to 13, the assembly ring 22 of the self-adsorption cover 20 is detachably mounted to the negative pressure forming part 12. This allows the self-adsorption cover 20 to be easily cleaned, improving the hygiene of the breast pump. Simultaneously, mothers can choose a suitable self-adsorption cover 20 to meet their individual needs. For example, self-adsorption covers 20 of different materials, sizes, and shapes can be selected and mounted on the same negative pressure forming part 12.

As shown in FIGS. 3 to 13, the outer diameter of the assembly ring 22 of the self-adsorption cover 20 is the same as the inner diameter of the negative pressure forming part 12, so that the assembly ring 22 of the self-adsorption cover 20 can be reliably mounted on the negative pressure forming part 12 based on the friction generated between the outer wall of the assembly ring 22 and the inner wall of the negative pressure forming part 12.

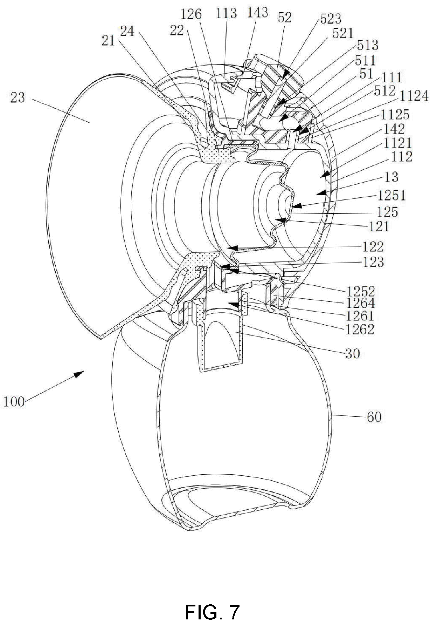

Referring to FIGS. 7, 8, and 12, the assembly ring 22 of the self-adsorption cover 20 is extended through the assembly opening 122 of the negative pressure forming part 12 to the outside of the milk discharge channel 123 of the negative pressure forming part 12, so as to form a step structure. In this way, the sucked-out milk can be blocked by the assembly ring 22, preventing milk from flowing to the position of the horn cover 23, thereby preventing milk leakage from the contact position between the horn cover 23 and the breast. In other words, by allowing the assembly ring 22 of the self-adsorption cover 20 to extend through the assembly opening 122 of the negative pressure forming part 12 to the outside of the milk discharge channel 123 of the negative pressure forming part 12, the sucked-out milk is only allowed to be discharged through the milk discharge channel 123 of the negative pressure forming part 12.

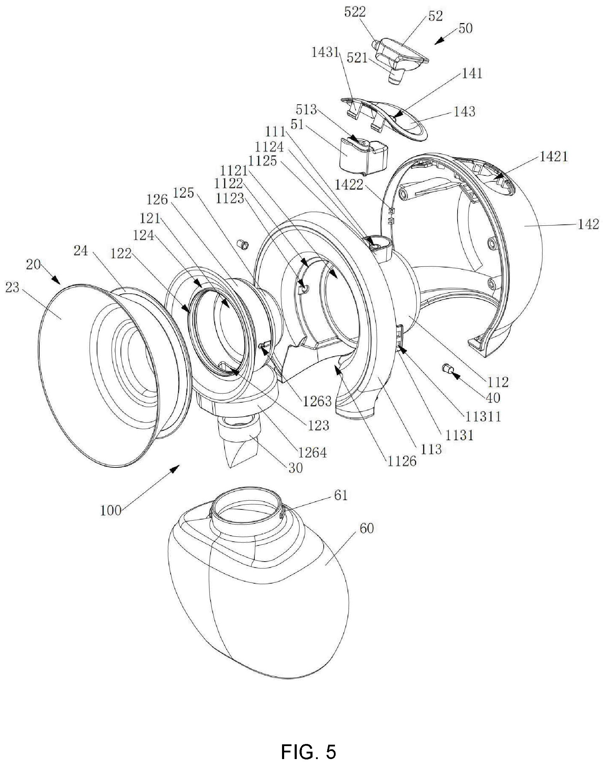

Referring to FIGS. 5 to 9, the self-adsorption cover 20 further comprises a limiting portion 24 which is extended integrally from the peripheral wall of the assembly ring 22 in a direction perpendicular to the extending direction of the assembly ring 22, and there is a gap between the limiting portion 24 and the shoulder ring 21. In this way, when the assembly ring 22 of the self-adsorption cover 20 is assembled at the assembly opening 122 of the negative pressure forming part 12, the limiting portion 24 restricts the assembly depth of the assembly ring 22 by abutting against the negative pressure forming part 12. Thus, on the one hand, the limiting portion 24 can ensure that there is a gap between the shoulder ring 21 and the negative pressure forming part 12, so that the shoulder ring 21 can deform when the assembly ring 22 changes with the position of the negative pressure forming unit 10. On the other hand, the limiting portion 24 can ensure that the assembly ring 22 extends through the assembly opening 122 of the negative pressure forming part 12 to the outside of the milk discharge channel 123 of the negative pressure forming part 12, so as to prevent the assembly ring 22 from covering the milk discharge channel 123 of the negative pressure forming part 12.

As shown in FIGS. 3 to 13, the limiting portion 24 is annular, and the distance d1 between the outer side of the limiting portion 24 and the central axis of the self-adsorption cover 20 is greater than the distance d2 between the outer side of the shoulder ring 21 and the central axis of the self-adsorption cover 20. Thus, from the top view of the self-adsorption cover 20 (the angle perpendicular to the central axis of the self-adsorption cover 20), the limiting portion 24 covers the shoulder ring 21, making the shoulder ring 21 visually invisible.

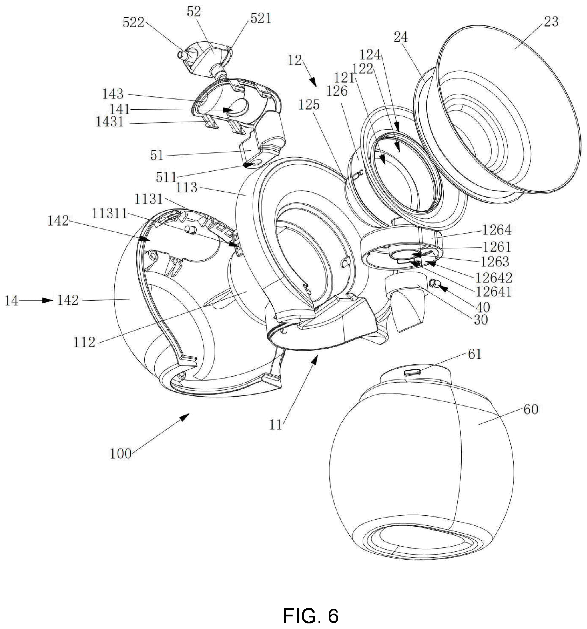

Referring to FIGS. 6 to 8, the negative pressure forming part 12 has an insert groove 124 surrounding the outside of the assembly opening 122. The limiting portion 24 of the self-adsorption cover 20 comprises an insert protrusion 241 whose shape and size match the shape and size of the insert groove 124 of the negative pressure forming part 12. The insert protrusion 241 of the limiting portion 24 is fitted into the insert groove 124 of the negative pressure forming part 12. Based on the frictional force generated between the inner wall of the negative pressure forming part 12 for forming the insert groove 124 and the outer wall of the insert protrusion 241 of the limiting portion 24, the assembly ring 22 of the self-adsorption cover 20 can be reliably assembled into the negative pressure forming part 12.

In this specific example of the self-adsorption milk pumping apparatus 100 shown in FIGS. 3 to 13, the insert groove 124 of the negative pressure forming part 12 is annular and surrounds the assembly opening 122. Correspondingly, the insert protrusion 241 of the limiting portion 24 of the self-adsorption cover 20 is annular and surrounds the assembly ring 22. Thus, when installing the self-adsorption cover 20 onto the negative pressure forming part 12, it is not necessary to pay attention to the installation direction of the self-adsorption cover 20.

Referring again to FIGS. 3 to 13, the supporting housing 11 comprises a housing body 112 and a horn body 113 integrally extended outward from one end of the housing body 112. The housing body 112 has a mounting cavity 1121 and a mounting opening 1122 communicated with the mounting cavity 1121. The air exchange channel 111 is disposed in the housing body 112 and is communicated to the mounting cavity 1121 of the housing body 112. The negative pressure forming part 12 comprises a deformable portion 125 and a frame portion 126. The milk suction chamber 121, the mounting opening 122, and the milk discharge channel 123 of the negative pressure forming part 12 are formed in the deformable portion 125. The deformable portion 125 has a connecting end 1251 and a deformable end 1252 opposite to the connecting end 1251. The frame portion 126 is connected to the connecting end 1251 of the deformable portion 125. The frame portion 126 of the negative pressure forming part 12 is installed in the mounting opening 1122 of the housing body 112 of the supporting housing 11. The deformable portion 125 extends through the mounting opening 1122 of the housing body 112 to the mounting cavity 1121. The negative pressure chamber 13 is formed between the deformable portion 125 and the housing body 112.

When the breast pump main unit 200 is in operation, the air in the negative pressure chamber 13 of the negative pressure forming unit 10 is discharged through the air exchange channel 111 of the supporting housing 11 and the air tube 300 to reduce the pressure in the negative pressure chamber 13. At this time, the one-way valve 30 closes the milk discharge channel 123 of the negative pressure forming part 12, and the deformable end 1252 of the deformable portion 125 deforms toward the end closer to the housing body 112 and away from the horn body 113 to increase the space of the milk suction chamber 121 of the negative pressure forming part 12, thereby increasing the pressure in the milk suction chamber 121. As the force decreases, air from the external environment enters the negative pressure chamber 13 of the negative pressure forming unit 10 through the airway 300 and the air exchange channel 111 of the supporting housing 11. The pressure in the negative pressure chamber 13 increases. At this time, the one-way valve 30 opens the milk discharge channel 123 of the negative pressure forming part 12 to discharge milk. The deformable end 1252 of the deformable portion 125 deforms toward the end away from the housing body 112 and away from the horn body 113, so as to reduce the space of the milk suction chamber 121 of the negative pressure forming part 12, resulting in an increase in the pressure of the milk suction chamber 121.

It is worth noting that the manner in which the frame portion 126 is attached to the connecting end 1251 of the deformable portion 125 is not limited in the self-adsorption milk pumping apparatus 100 of the present invention. For example, in a specific example of the self-adsorption milk pumping apparatus 100 of the present invention, the deformable portion 125 and the frame portion 126 are made separately, and glue is used to bond the frame portion 126 to the connecting end 1251 of the deformable portion 125, thereby the frame portion 126 is attached to the connecting end 1251 of the deformable portion 125. In another specific example of the self-adsorption milk pumping apparatus 100 of the present invention, firstly, the deformable portion 125 is made, and secondly, the frame portion 126 is integrally attached to the deformable portion 125 by a secondary overmolding process, thereby the frame portion 126 is attached to the connecting end 1251 of the deformable portion 125. In another specific example of the self-adsorption type breast pumping device 100 of the present invention, firstly, the frame portion 126 is manufactured, and secondly, the connecting end 1251 of the deformable portion 125 is integrally bonded to the frame portion 126 by a secondary coating process, so that the frame portion 126 is bonded to the connecting end 1251 of the deformable portion 125.

As shown in FIGS. 3 to 13, the frame portion 126 of the negative pressure forming part 12 surrounds the connecting end 1251 of the deformable portion 125, such that the frame portion 126 enables the negative pressure forming part 12 to be reliably mounted on the supporting housing 11.

Referring to FIGS. 5 to 8, the bottom of the frame portion 126 of the negative pressure forming part 12 is provided with an internal mounting arm 1261 and an arm body channel 1262. The arm body channel 1262 passes through the internal mounting arm 1261 and communicates with the milk discharge channel 123 of the negative pressure forming part 12. The one-way valve 30 is installed on the internal mounting arm 1261 of the frame portion 126 to set the one-way valve 30 in the negative pressure forming part 12.

Preferably, the one-way valve 30 is a duckbill valve. As the pressure in the milk suction chamber 121 of the negative pressure forming part 12 changes, the one-way valve 30 can switch between an open state and a closed state. When the deformable end 1252 of the deformable portion 125 deforms toward the end away from the horn body 113 near the housing body 112, causing the pressure in the milk suction chamber 121 to decrease, the one-way valve 30 closes the milk discharge channel 123 of the negative pressure forming part 12. When the deformable end 1252 of the deformable portion 125 deforms toward the end away from the horn body 113 away from the housing body 112, causing the pressure in the milk suction chamber 121 to increase, the one-way valve 30 opens the milk discharge channel 123 of the negative pressure forming part 12.

In one example of the self-adsorption milk pumping apparatus 100 of the present invention shown in FIGS. 3 to 13, the negative pressure forming part 12 is detachably mounted to the supporting housing 11, so that the negative pressure forming part 12 can be cleaned after being removed from the supporting housing 11, thereby improving the hygiene of the self-adsorption milk pumping apparatus 100.

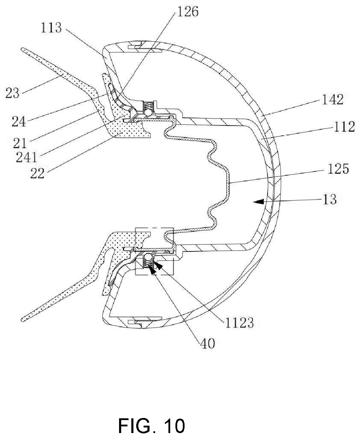

Referring again to FIGS. 10 to 13, the self-adsorption milk pumping apparatus 100 further comprises at least one ball head plunger 40. The ball head plunger 40 comprises a cylindrical portion 41, a compression spring 42, and a ball head portion 43. The cylindrical portion 41 has a cylindrical cavity 411 and an opening 412 communicated with the cylindrical cavity 411. The compression spring 42 is compressibly disposed in the cylindrical cavity 411 of the cylindrical portion 41. The ball head portion 43 is movably disposed in the opening 412 of the cylindrical portion 41, and the bottom of the ball head portion 43 abuts against the compression spring 42, while the top of the ball head portion 43 is protruded from the cylindrical portion 41. The cylindrical portion 41 of the ball head plunger 40 is fixedly mounted to the housing body 112 of the supporting housing 11. The ball head portion 43 is protruded from the mounting cavity 1121 of the housing body 112. The outer wall of the frame portion 126 of the negative pressure forming part 12 has at least one retaining groove 1263. When the negative pressure forming part 12 is installed on the supporting housing 11, the outer wall of the frame portion 126 pushes the ball head portion 43 toward the bottom of the cylindrical portion 41, so that the ball head portion 43 compresses the compression spring 42 to cause the compression spring 42 to deform and accumulate elastic potential energy. When the negative pressure forming part 12 is installed in place, the compression spring 42 pushes the ball head portion 43 away from the bottom of the cylindrical portion 41, so that the ball head portion 43 protrudes into the retaining groove 1263 of the frame portion 126. Based on the frictional force generated between the peripheral wall of the frame portion 126 that forms the retaining groove 1263 and the outer surface of the ball head portion 43, the ball head plunger 40 reliably installs the negative pressure forming part 12 onto the supporting housing 11. Accordingly, when removing the negative pressure forming part 12 from the supporting housing 11, as long as the pulling force of the negative pressure forming part 12 is greater than the frictional force generated between the peripheral wall of the frame portion 126 that forms the retaining groove 1263 and the outer surface of the ball head portion 43, the ball head portion 43 can be disengaged from the retaining groove 1263 of the frame portion 126.

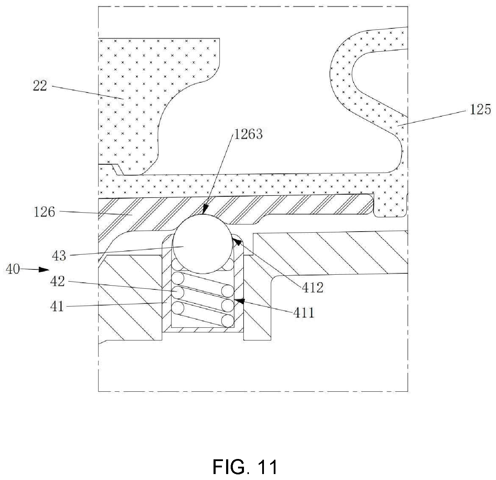

It is worth mentioning that the manner in which the cylindrical portion 41 of the ball head plunger 40 is fixedly mounted to the housing body 112 of the supporting housing 11 is not limited in the self-adsorption milk pumping apparatus 100 of the present invention. For example, in this specific example of the self-adsorption milk pumping apparatus 100 of the present invention, referring to FIGS. 10 and 11, the housing body 112 of the supporting housing 11 has at least one mounting hole 1123, and the cylindrical portion 41 of the ball head plunger 40 is mounted in the mounting hole 1123 of the housing body 112 to fix the cylindrical portion 41 to the housing body 112.

In a specific example of the self-adsorption milk pumping apparatus 100 of the present invention, the housing body 112 of the supporting housing 11 has two mounting holes 1123, which are located on two sides of the mounting cavity 1121. There are two ball head plungers 40, and the cylindrical portion 41 of each ball head plunger 40 is fixedly mounted in each mounting hole 1123 of the housing body 112. Thus, a ball head plunger 40 is provided on each opposite side of the mounting cavity 1121 of the housing body 112. Correspondingly, a retaining groove 1263 is provided on each opposite side of the frame portion 126 of the negative pressure forming part 12, so that when the negative pressure forming part 12 is installed on the housing body 112 of the supporting housing 11, the ball head portions 43 of the two ball head plungers 40 are respectively held in the two retaining grooves 1263 of the frame portion 126. In this way, the negative pressure forming part 12 can be reliably installed in the mounting opening 1122 of the housing body 112. In this way, the frame portion 126 and the housing body 112 can squeeze a portion of the deformable portion 125, so that the portion of the deformable portion 125 forms a gap between the frame portion 126 and the housing body 112 by deforming, thus ensuring the sealing of the negative pressure chamber 13.

In one example of the self-adsorption milk pumping apparatus 100 of the present invention shown in FIGS. 3 to 13, the self-adsorption milk pumping apparatus 100 comprises an airway unit 50 which comprises an air guide base 51 and a reversing base 52. The air guide base 51 has a laterally extending first air passage 511 and a bottom insertion hole 512 and a top insertion hole 513 respectively connected to the first air passage 511. The top of the housing body 112 of the supporting housing 11 has a housing insertion arm 1124, and the opening of the air exchange channel 111 is formed on the end face of the housing insertion arm 1124. The air guide base 51 is disposed on the top of the housing body 112, such that the housing insertion arm 1124 is inserted into the bottom insertion hole 512. The reversing base 52 comprises a bottom insert arm 521, a side insert arm 522, and has a second air passage 523. One opening of the second air passage 523 is formed on the end face of the bottom insert arm 521, and the other opening is formed on the end face of the side insert arm 522. The bottom insert arm 521 of the reversing base 52 is inserted into the top insertion hole 513 of the air guide base 51, so that the negative pressure chamber 13 of the negative pressure forming unit 10, the first air passage 511 of the air guide base 51, and the second air passage 523 of the reversing base 52 are sequentially connected.

In this example of the self-adsorption milk pumping apparatus 100 shown in FIGS. 3 to 13, by providing the air guide base 51 and the reversing base 52 on the top of the housing body 112 of the supporting housing 11, the part of the self-adsorption milk pumping apparatus 100 used to connect the air tube 300 (i.e., the side insert arm 522 of the reversing base 52) can be positioned reasonably, avoiding this part occupying a large space. In other words, the arrangement of the air guide base 51 and the reversing base 52 of the airway unit 50 enables the miniaturization of the self-adsorption milk pumping apparatus 100. Thus, after the mother attaches the self-adsorption milk pumping apparatus 100 to her breast, if she needs to wear a bra, the self-adsorption milk pumping apparatus 100 will not excessively push the bra outwards. At the same time, the pressure exerted on the breast by the bra through the self-adsorption milk pumping apparatus 100 will also be reduced, thereby improving the comfort of the mother when pumping milk using the self-adsorption milk pumping apparatus 100.

In this example of the self-adsorption milk pumping apparatus 100 shown in FIGS. 3 to 13, the bottom insert arm 521 of the reversing base 52 is detachably inserted into the top insertion hole 513 of the air guide base 51. This allows the mother to select the orientation of the side insert arm 522 of the reversing base 52 as needed. For example, the mother can allow the bottom insert arm 521 of the reversing base 52 to be inserted into the top insertion hole 513 of the air guide base 51 with the side insert arm 522 of the reversing base 52 facing left, or the bottom insert arm 521 of the reversing base 52 to be inserted into the top insertion hole 513 of the air guide base 51 with the side insert arm 522 of the reversing base 52 facing right. In this way, the mother does not need to distinguish between the two self-adhesive breast pumps 100 during the assembly of the breast pump.

In this example of the self-adsorption milk pumping apparatus 100 shown in FIGS. 3 to 13, the negative pressure forming unit 10 further comprises an outer casing 14 and has a receiving cavity 15. The outer casing 14 has a positioning hole 141. The outer casing 14 is fixedly mounted to the outer edge of the horn body 113 of the supporting housing 11 to form the receiving cavity 15 between the supporting housing 11 and the outer casing 14. The positioning hole 141 of the outer casing 14 is communicated with the receiving cavity 15. The air guide base 51 has a top boss 514, and the opening of the top insertion hole 513 is formed on the end face of the top boss 514. The air guide base 51 is received in the receiving cavity 15 of the negative pressure forming unit 10, and the top boss 514 of the air guide base 51 is extended to the positioning hole 141 of the outer casing 14. It is understood that, since the housing insert arm 1124 of the housing body 112 of the supporting housing 11 is inserted into the bottom insertion hole 512 of the air guide base 51 and the top boss 514 of the air guide base 51 is extended to the positioning hole 141 of the outer casing 14, the air guide base 51 is stably housed in the receiving cavity 15 of the negative pressure forming unit 10. Whether the bottom insert arm 521 of the reversing base 52 is inserted into the top insertion hole 513 of the air guide base 51 or the bottom insert arm 521 of the reversing base 52 is pulled out from the top insertion hole 513 of the air guide base 51, the air guide base 51 will not shift.

Referring to FIGS. 7 and 8, the top of the housing body 112 of the supporting housing 11 has a positioning wall 1125. The positioning wall 1125 surrounds the bottom of the air guide base 51, and the inner wall of the positioning wall 1125 fits against the outer wall of the air guide base 51. In this way, before the outer casing 14 is assembled to the supporting housing 11, the positioning wall 1125 of the housing body 112 can pre-position the air guide base 51, thereby facilitating the assembly of the outer casing 14 to the supporting housing. During assembly, the positioning wall 1125 of the housing body 112 can prevent the air guide base 51 from being misaligned, thus improving assembly efficiency. On the other hand, when the bottom insert arm 521 of the reversing base 52 is inserted into the top insertion hole 513 of the air guide base 51 or when the bottom insert arm 521 of the reversing base 52 is pulled out from the top insertion hole 513 of the air guide base 51, the positioning wall 1125 of the housing body 112 can prevent the air guide base 51 from shifting.

Referring again to FIGS. 5 to 8, the outer casing 14 comprises an outer shell 142 and an anti-detachment cover 143. The positioning hole 141 is formed in the anti-detachment cover 143. The outer shell 142 has an outer shell channel 1421. The outer shell 142 is fixedly installed on the outer edge of the horn body 113 of the supporting housing 11. The position of the top boss 514 of the air guide base 51 corresponds to the position of the outer shell channel 1421 of the outer shell 142. The anti-detachment cover 143 is fixedly installed in the outer shell channel 1421 of the outer shell 142 by inserting the top boss 514 of the air guide base 51 into the positioning hole 141 of the outer casing 14. In this way, the supporting housing 11, the air guide base 51, and the outer casing 14 can be easily installed, and misalignment of the air guide base 51 is avoided.

It is worth mentioning that the manner in which the outer shell 142 is fixedly mounted to the horn body 113 of the supporting housing 11 is not limited in the self-adsorption milk pumping apparatus 100 of the present invention. For example, in this specific example of the self-adsorption milk pumping apparatus 100 of the present invention, referring to FIG.5, the rear side of the horn body 113 of the supporting housing 11 has a plurality of locking arms 1131, the locking arms 1131 having locking holes 11311, and the inner wall of the outer shell 142 having a plurality of locking protrusions 1422. These locking arms 1131 of the horn body 113 extend into the interior of the outer shell 142, and these locking protrusions 1422 of the outer shell 142 are respectively locked into the locking holes 11311 of the locking arms 1131, so that the outer shell 142 is fixedly mounted to the horn body 113 of the supporting housing 11. Optionally, in other specific examples of the self-adsorption milk pumping apparatus 100 of the present invention, the locking arm 1131 may be provided on the outer shell 142, and the locking protrusion 1422 may be provided on the horn body 113, so that the locking arms 1131 of the outer shell 142 extend into the interior of the horn body 113, and the locking protrusions 1422 of the horn body 113 are respectively locked into the locking holes 11311 of the locking arms 1131 of the outer shell 142, so that the outer shell 142 is fixedly mounted on the horn body 113 of the supporting housing 11.

It is worth mentioning that the manner in which the anti-detachment cover 143 is fixedly installed in the outer shell channel 1421 of the outer shell 142 is not limited in the self-adsorption milk pumping apparatus 100 of the present invention. For example, in this specific example of the self-adsorption milk pumping apparatus 100 of the present invention, referring to FIGS. 5 to 7, the anti-detachment cover 143 has a plurality of hook arms 1431 on opposite sides, the shape of the anti-detachment cover 143 matches the shape of the outer shell channel 1421 of the outer shell 142, and the size of the anti-detachment cover 143 is slightly larger than the size of the outer shell channel 1421 of the outer shell 142. These hook arms 1431 of the anti-detachment cover 143 extend from the outside to the inside of the outer shell 142 through the outer shell channel 1421 of the outer shell 142 to fix the anti-detachment cover 143 in the outer shell channel 1421 of the outer shell 142, and the anti-detachment cover 143 seals the outer shell channel 1421 of the outer shell 142.

In this example of the self-adsorption milk pumping apparatus 100 shown in FIGS. 3 to 13, the bottom of the housing body 112 of the supporting housing 11 has a mounting notch 1126, and the bottom of the frame portion 126 of the negative pressure forming part 12 has an outer mounting arm 1264 which surrounds the internal mounting arm 1261 and can be held in the mounting notch 1126 of the housing body 112. The self-adsorption milk pumping apparatus 100 further comprises a bottle 60, which is mounted on the frame portion 126. The outer mounting arm 1264 of the frame portion 126 is configured such that: on the one hand, the sucked milk can be collected into the bottle 60; on the other hand, the frame portion 126 is held above the portion of the housing body 112 that forms the mounting notch 1126, and the bottle 60 is held below it. Thus, the frame portion 126 and the bottle 60 clamp the portion of the housing body 112 that forms the mounting notch 1126 on opposite sides, thereby further ensuring that the negative pressure forming part 12 is reliably and stably mounted on the supporting housing 11.

Referring to FIGS. 5 and 6, the outer mounting arm 1264 of the frame portion 126 of the negative pressure forming part 12 has at least one locking groove 12641 extending in the circumferential direction and at least one guide groove 12642 extending in the height direction and communicating with one end of the locking groove 12641. The bottle mouth of the baby bottle 60 has at least one locking protrusion 61. The locking protrusion 61 of the baby bottle 60 can slide into the locking groove 12641 through the guide groove 12642 of the outer mounting arm 1264 of the frame portion 126 to mount the baby bottle 60 to the outer mounting arm 1264 of the frame portion 126.

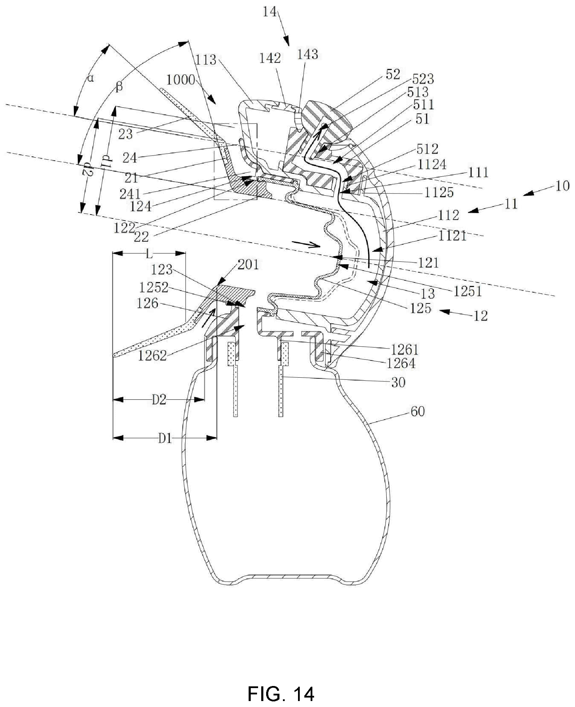

FIG. 14 shows a modified example of the self-adsorption milk pumping apparatus 100 of the present invention. Unlike the self-adsorption milk pumping apparatuss 100 shown in FIGS. 3 to 13, in this specific example of the self-adsorption milk pumping apparatus 100 shown in FIG. 14, the shoulder ring 21 and the assembly ring 22 of the self-adsorption cover 20 are rigid, while the horn cover 23 is flexible. Thus, on the one hand, the shoulder ring 21 can maintain the shape of the horn cover 23, preventing the horn cover from deformation. On the other hand, when the self-adsorption milk pumping apparatus 100 moves downward due to its own weight, the shoulder ring 21 can slightly hold the horn cover 23, providing an upwardly tilted support force for the negative pressure forming unit 10, so that the self-adsorption milk pumping apparatus 100 can be stably adsorbed onto the breast. Furthermore, the assembly ring 22 and the shoulder ring 21 can directly transmit the vibration of the negative pressure forming unit 10 to the root of the breast, which is conducive to the extraction of hindmilk, thereby improving the breast pumping effect.

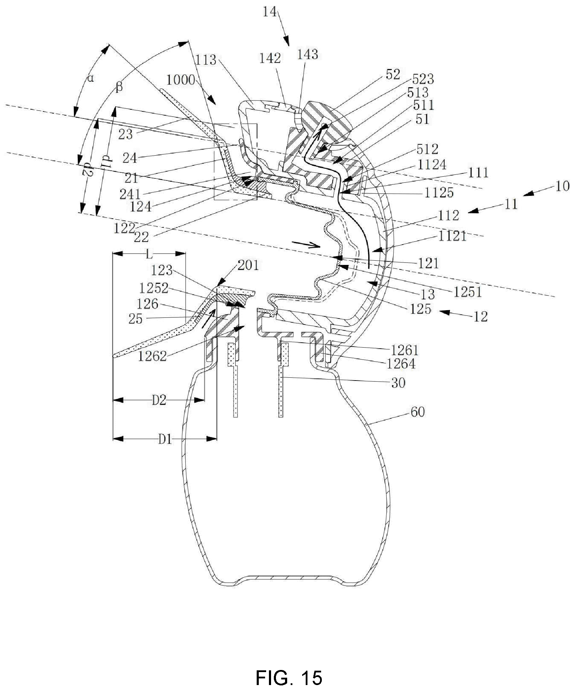

FIG. 15 shows a modified example of the self-adsorption milk pumping apparatus 100 of the present invention. Unlike the self-adsorption milk pumping apparatus 100 shown in FIG. 14, in this specific example of the self-adsorption milk pumping apparatus 100 shown in FIG. 15, the shoulder ring 21, the assembly ring 22, and the horn cover 23 of the self-adsorption cover 20 are all flexible. At the same time, the self-adsorption cover 20 also comprises a stabilizing part 25 which is disposed on the outside of the assembly ring 22 and the shoulder ring 21 to maintain the shape of the horn cover 23 and prevent the horn cover 23 from deforming. At the same time, the arrangement of the stabilizing part 25 is conducive to the stable adhesion of the self-adsorption milk pumping apparatus 100 to the breast and the direct transmission of vibration to the root of the breast, which is conducive to the extraction of hindmilk.

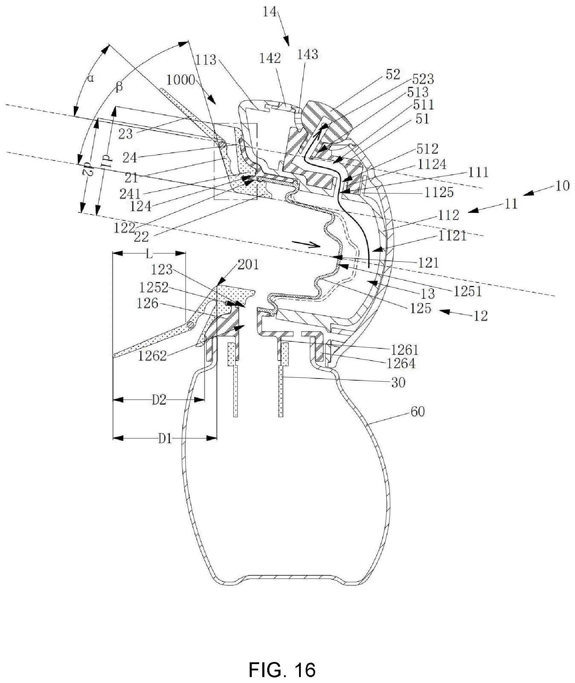

FIG. 16 shows a modified example of the self-adsorption milk pumping apparatus 100 of the present invention. Unlike the self-adsorption milk pumping apparatus 100 shown in FIGS. 3 to 13, in this specific example of the self-adsorption milk pumping apparatus 100 shown in FIG. 16, the stabilizing part 25 is provided at the connection position of the shoulder ring 21 and the horn cover 23 to maintain the shape of the horn cover 23 and prevent the horn cover 23 from deforming.

Preferably, the stabilizing portion 25 is annular and is enclosed inside the self-adsorption cover 20. For example, an insert injection molding process can be used to manufacture the self-adsorption cover 20, so that the stabilizing portion 25 is enclosed inside the self-adsorption cover 20.

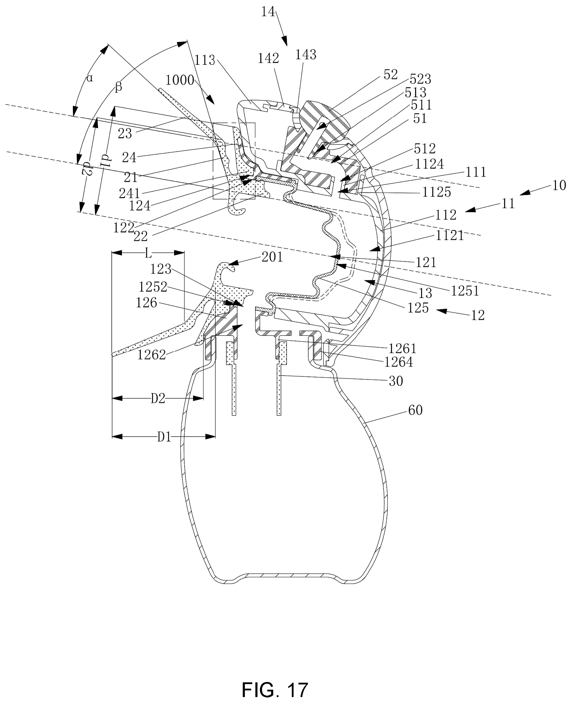

FIG. 17 shows a modified example of the self-adsorption milk pumping apparatus 100 of the present invention. Unlike the self-adsorption milk pumping apparatus 100 shown in FIGS. 3 to 13, in this specific example of the self-adsorption milk pumping apparatus 100 shown in FIG. 17, the abutment portion 201 of the self-adsorption cover 20 is extended inward and backward from the connection between the shoulder ring 21 and the assembly ring 22. When the self-adsorption milk pumping apparatus 100 is self-attached to the breast, the abutment portion 201 of the self-adsorption cover 20 abuts against the areola. During the pumping process, when the pressure of the milk suction chamber 121 of the negative pressure forming part 12 decreases, the areola is pulled towards the milk suction chamber 121 of the negative pressure forming part 12. The abutment portion 201 provides resistance to prevent the areola from deforming toward the milk suction chamber 121 of the negative pressure forming part 12. At this time, the areola is compressed under pressure to improve the pumping efficiency and massage the areola. Understandably, the abutment portion 201 of the self-adsorption cover 20, while providing resistance, deforms towards the milk suction chamber 121 of the negative pressure forming part 12, so that the areola receives resistance while avoiding hard squeezing, thereby improving comfort. Furthermore, a gap exists between the abutment portion 201 of the self-adsorption cover 20 and the nipple, allowing the nipple to be suspended, thus preventing pressure damage to the nipple during milk suction.

Those skilled in the art should understand that the embodiments of the present invention described above and shown in the accompanying drawings are merely examples and do not limit the present invention. The objectives of the present invention have been fully and effectively achieved. The functions and structural principles of the present invention have been demonstrated and explained in the embodiments, and any variations or modifications may be made to the implementation of the present invention without departing from the stated principles.

## Claims

1. A self-adsorption milk pumping apparatus, **characterized in that** comprising:
a one-way valve;
a self-adsorption cover; and
a negative pressure forming unit having a negative pressure chamber, an air exchange channel, a milk suction chamber, an assembly opening, and a milk discharge channel, wherein the air exchange channel is communicated to the negative pressure chamber, the assembly opening and the milk discharge channel are respectively communicated to the milk suction chamber, the negative pressure forming unit comprises a deformable component, and the milk suction chamber and the negative pressure chamber are separated by the deformable component of the negative pressure forming unit, wherein the self-adsorption cover is installed in the assembly opening of the negative pressure forming unit, and the one-way valve is provided in the negative pressure forming unit to selectively open and close the milk discharge channel.

2. The self-adsorption milk pumping apparatus according to claim 1, wherein the self-adsorption cover has a horn cover which is deformable, and from a side view, an active space is formed between the horn cover and the negative pressure forming unit to allow the horn cover to flip towards the negative pressure forming unit.

3. The self-adsorption milk pumping apparatus according to claim 1, wherein the self-adsorption cover comprises a horn cover which is deformable and an abutment portion, the horn cover is arranged to cover a portion of a breast, the abutment portion is arranged to abut an areola, wherein a distance (D1) between the abutment portion and an outer edge of the horn cover is greater than a distance (D2) between a portion of the negative pressure forming unit arranged to define the assembly opening and the outer edge of the horn cover.

4. The self-adsorption milk pumping apparatus according to claim 2, wherein the self-adsorption cover comprises an abutment portion for abutting an areola, wherein a distance (D1) between the abutment portion and an outer edge of the horn cover is greater than a distance (D2) between a portion of the negative pressure forming unit arranged to define the assembly opening and the outer edge of the horn cover.

5. The self-adsorption milk pumping apparatus according to claim 1, wherein the self-adsorption cover comprises a shoulder ring, an assembly ring, and a horn cover which is deformable, wherein the assembly ring is extended integrally to one side from an inner edge of the shoulder ring, and the horn cover is extended integrally in an opposite direction from an outer edge of the shoulder ring.

6. The self-adsorption milk pumping apparatus according to claim 2, wherein the self-adsorption cover further comprises a shoulder ring and an assembly ring, wherein the assembly ring is extended integrally to one side from an inner edge of the shoulder ring, and the horn cover is extended integrally in an opposite direction from an outer edge of the shoulder ring.

7. The self-adsorption milk pumping apparatus according to claim 6, wherein the self-adsorption cover comprises an abutment portion located at a connection between the assembly ring and the shoulder ring for abutting an areola, wherein a distance (D1) between the abutment portion and an outer edge of the horn cover is greater than a distance (D2) between a portion of the negative pressure forming unit arranged to define the assembly opening and the outer edge of the horn cover.

8. The self-adsorption milk pumping apparatus according to claim 5, wherein the self-adsorption cover comprises a stabilizing part which is disposed at a connection position between the shoulder ring and the horn cover to maintain a shape of the horn cover.

9. The self-adsorption milk pumping apparatus according to claim 5, wherein the horn cover is flexible, the shoulder ring and the assembly ring are rigid.

10. The self-adsorption milk pumping apparatus according to claim 5, wherein the self-adsorption cover comprises a stabilizing part disposed on the outside of the assembly ring and the shoulder ring to maintain the shape of the horn cover.

11. The self-adsorption milk pumping apparatus according to one of claims 1-7, wherein the self-adsorption cover is extended through the assembly opening of the negative pressure forming unit to an outside of the milk discharge channel to form a step.

12. The self-adsorption milk pumping apparatus according to claim 5, 6 or 7, wherein the shoulder ring is extended outward and forward from the assembly ring in a direction substantially perpendicular to the assembly ring, wherein the horn cover is extended outward and forward from the shoulder ring in a direction substantially horizontal to the assembly ring.

13. The self-adsorption milk pumping apparatus according to claim 5, 6 or 7, wherein the self-adsorption cover comprises a limiting portion that is extended integrally from a peripheral wall of the assembly ring in a direction perpendicular to an extending direction of the assembly ring, and the limiting portion is spaced part from the shoulder ring.

14. The self-adsorption milk pumping apparatus according to claim 13, wherein a distance (d1) between an outer side of the limiting portion and a central axis of the self-adsorption cover is greater than a distance (d2) between an outer side of the shoulder ring and the central axis of the self-adsorption cover.

15. The self-adsorption milk pumping apparatus according to one of claims 2-7, wherein a hardness of the horn cover of the self-adsorption cover is in the range of 10A to 50A.

16. The self-adsorption milk pumping apparatus according to one of claims 2-7, wherein a projected length (L) of the horn cover of the self-adsorption cover in a horizontal plane ranges from 12 mm to 30 mm.

17. The self-adsorption milk pumping apparatus according to one of claims 2-7, wherein a surface roughness of an inner wall of the horn cover ranges from Ra0.016um to Ra0.063um.

18. The self-adsorption milk pumping apparatus according to one of claims 2-7, wherein an angle α between an extending direction of an inner wall of the horn cover of the self-adsorption cover and a central axis of the self-adsorption cover ranges from 15° to 40°.

19. The self-adsorption milk pumping apparatus according to one of claims 2-7, wherein an extension direction of an inner wall of the horn cover of the self-adsorption cover and a central axis of the self-adsorption cover define an angle α, and an extension direction of an inner wall of the shoulder ring and the central axis of the self-adsorption cover define an angle β, wherein the angle β is greater than the angle α.

20. The self-adsorption milk pumping apparatus according to one of claims 1-7, wherein the negative pressure forming unit comprises a supporting housing and a negative pressure forming part which is installed on the supporting housing, wherein the negative pressure chamber is formed between the supporting housing and the negative pressure forming part, the air exchange channel is provided on the supporting housing, the milk suction chamber, the assembly opening and the milk discharge channel are provided on the negative pressure forming part, wherein the self-adsorption cover and the one-way valve are respectively installed on the negative pressure forming part.

21. The self-adsorption milk pumping apparatus according to claim 20, wherein the supporting housing comprises a housing body and a horn body integrally extended outward from one end of the housing body, wherein the housing body has a mounting cavity and a mounting opening communicated with the mounting cavity, wherein the negative pressure forming part comprises a deformable portion and a frame portion, the deformable portion has a connecting end and a deformable end opposite to the connecting end, the milk suction chamber, the assembly opening, and the milk discharge channel are formed in the deformable portion, the frame portion is connected to the connecting end of the deformable portion, the frame portion of the negative pressure forming part is mounted to the mounting opening of the housing body, the deformable portion is extended through the mounting opening to the mounting cavity, the negative pressure chamber is formed between the deformable portion and the housing body, the deformable portion is the deformable component of the negative pressure forming unit for separating the negative pressure chamber and the milk suction chamber.

22. A breast pump,**characterized in that** comprising:
a breast pump main unit;
at least one air tube; and
at least one self-adsorption milk pumping apparatus according to one of claims 1-21, wherein the air tube connects the breast pump main unit and the air exchange channel of the negative pressure forming unit.
